# EUROPÄISCHE PATENTSCHRIFT

(11) **EP 0 793 974 B1**
(45) Veröffentlichungstag und Bekanntmachung des Hinweises auf die Patenterteilung: **15.09.2004**
(21) Anmeldenummer: 97250058.1
(22) Anmeldetag: 04.03.1997
(51) Int. Cl.: A61N 1/05, A61N 1/02, A61N 1/375

(54) **Implantierbares Elektrostimulationsgerät und Elektrodenleitung**
Implantable stimulation device and electrode system for same
Appareil de stimulation implantable et système d'électrodes correspondant

(30) Priorität: 04.03.1996 DE 19609367
(43) Veröffentlichungstag der Anmeldung: 10.09.1997
(73) Patentinhaber: BIOTRONIK Mess- und Therapiegeräte GmbH & Co Ingenieurbüro Berlin, 12359 Berlin (DE)
(72) Erfinder: Heil, Horst F., Dr., 95138 Bad Steben (DE)
(74) Vertreter: Eisenführ, Speiser & Partner

(56) Entgegenhaltungen:
- EP-A- 0 484 107
- DE-A- 2 845 175
- US-A- 3 760 332
- US-A- 4 112 953
- US-A- 4 764 132
- US-A- 5 088 942

## Beschreibung

Die Erfindung betrifft ein implantierbares Elektrostimulationsgerät, beispielsweise einen Herzschrittmacher oder Defibrillator, der im Oberbegriff des Anspruchs 1 angegebenen Art und eine Elektrodenleitung gemäß dem Oberbegriff des Anspruchs 7.

Aus der DE-A-3 311 510 ist eine Anordnung zum Verbinden des proximalen Endes einer bipolaren Elektrodenleitung mit einem Herzschrittmacher bekannt, wobei die Elektrodenleitung einen vorderen Stiftkontakt und einen in distaler Richtung dahinterliegenden Ringkontakt aufweist. Die proximalen Stiftkontakte der Elektrodenleitung werden bei der Verbindung mit dem Herzschrittmacher in einen hohlzylindrischen, sogenannten Endblock eingebracht und dort mit einer Schraube in seiner Position fixiert. Die Stiftschraube in dem Endblock soll einerseits eine ausreichende elektrische Kontaktierung sichern und andererseits einen Schutz vor einer unerwünschten axialen Verschiebung des Leitungskontakts innerhalb des Endblocks bieten, damit sich das proximale Ende der bipolaren Elektrodenleitung nicht von dem Herzschrittmacher lösen kann. Der in distaler Richtung hinter dem Stiftkontakt vorgesehene zweite, als Ringkontakt ausgebildete Anschluss der Elektrodenleitung ist zwecks Kontaktierung in eine Buchse eingeschoben, welche an ihrer inneren Oberfläche drei Einschnitte aufweist. In diesen Einschnitten ist ein im wesentlichen kreisförmig gebogener Draht unter einer mechanischen Vorspannung derart eingesetzt, dass der Ringkontakt an drei Punkten elektrisch mit dem Draht verbunden ist, wenn sich der Stiftkontakt der Elektrodenleitung in dem für ihn vorgesehenen Endblock befindet.

Diese Lösung weist den Nachteil auf, dass die Verschraubung und die Verwendung eines in eine Buchse eingesetzten, ringförmig gebogenen Drahtes keine großflächige Kontaktierung sichern kann und zudem nur eine ungenügende Sicherheit gegen unbeabsichtigtes Lösen der Verbindung bei einer axialen Belastung der Elektrodenleitung bietet.

In US-A-4 583 543 wird eine Anordnung zur Verbindung einer bipolaren Elektrodenleitung mit einem Herzschrittmacher vorgeschlagen, bei der zum Anschluss einer dünneren als der ursprünglich vorgesehenen Elektrodenleitung im Kopfteil des Herzschrittmachers ein Adapter vorgesehen ist. Die Verbindung zwischen Adapter und Elektrodenleitung wird über einen Reibschluss hergestellt, welcher allein durch eine mehrfache, im wesentlichen lediglich punktförmige Kontaktierung auf einer einzigen Umfangslinie des Kontakts der Elektrodenleitung entsteht. Die Sicherung gegen ein Lösen der Verbindung bei einer axialen Belastung der Elektrodenleitung erscheint als unzureichend.

Des weiteren ist aus EP-A-0 339 877 ein Anschlusssystem für einen Herzschrittmacher bekannt, bei welchem im Kopfteil des Schrittmachers zwei Metallzylinder mit innenliegenden Federdrähten zur Kontaktierung der Anschlüsse der Elektrodenleitung vorgesehen sind. Die Sicherung gegen Lösen der elektrischen Verbindung zwischen Elektrodenleitung und Herzschrittmacher bei mechanischer Belastung in axialer Richtung ist auch hier ungenügend, da dieser lediglich ein Reibschluss in im wesentlichen linienförmigen Kontaktbereichen entgegenwirkt. Ein Elektrostimulationsgerät gemäß dem Oberbegriff von Anspruch 1 und eine Elektrodenleitung gemäß dem Oberbegriff von Anspruch 7 sind aus DE 28 45 175 bekannt.

Ausgehend von diesem Stand der Technik liegt der Erfindung die Aufgabe zugrunde, ein Elektrostimulationsgerät der eingangs genannten Gattung und eine mit dem Elektrostimulationsgerät verbindbare Elektrodenleitung zu schaffen, die in einfacher Weise und schnell miteinander verbindbar sind und deren Verbindung zugleich eine hohe Sicherheit gegenüber axialem Zug an der Elektrodenleitung aufweist.

Diese Aufgabe wird hinsichtlich des Elektrostimulationsgerätes mit den Merkmalen des Anspruchs 1 und hinsichtlich der Elektrodenleitung mit den Merkmalen gemäß Anspruch 7 gelöst.

Die Erfindung schließt den Gedanken ein, an der Anschlussbuchse des Elektrostimulationsgerätes und/oder an mindestens einem der Kontakte am Elektrodenleitungsstecker fest angebracht eine Mehrzahl von Eingriffsbereichen zur relativ großflächigen elektrischen Kontaktierung und Erzeugung eines Formschlusses und vorzugsweise zusätzlich eines Kraftschlusses vorzusehen, wodurch einer im wesentlichen axial gerichteten Zugkraft ein hoher Widerstand entgegengesetzt wird.

Diese Kontakt- und Haltemittel in Form von Nocken- oder Vorsprungsbereichen sind bevorzugt gruppenweise in mehreren sich zueinander parallel und in axialer Reihung erstreckenden Ebenen angeordnet.

Zur Bildung der radial nach innen gerichteten Kontakt- und Haltemittel der Anschlussbuchse im Kopfteil des Herzschrittmachers ist entsprechend einer technologischen Variante der Anschlussbuchse eine in die Buchse eingesetzte Hülse mit brückenartigen, elastischen Vorsprungsbereichen vorgesehen, die speziell durch Prägen und Biegen aus einem flächigen, Federeigenschaften und bevorzugt hohe Leitfähigkeit aufweisenden Material gefertigt ist. Diese Hülse umschließt straff elastisch einen eingeführten herkömmlichen Stift- oder Ringkontakt einer Elektrodenleitung und erbringt durch die relativ große effektive Kontaktfläche und angemessenen Anpressdruck sowohl einen guten elektrischen Kontakt als auch eine hohen Widerstand gegen axiale Zugkräfte. Die Hülse mit brückenartigen, elastischen Vorsprungsbereichen ist nicht Teil der Erfindung. Eine ähnliche Vorrichtung ist in US 4 764 132 beschrieben. Anschlussbuchsen mit Federelementen zum Erzeugen eines hohen Widerstandes gegen axiale Zugkräfte sind auch in US 4 112 953 und US 3 760 332 beschrieben.

Bei der Erfindung sind zwischen mehreren in Umfangsrichtung gruppierten Vorsprüngen der Anschlussbuchse jeweils Ausnehmungen vorhanden, in die beim Einstecken der Elektrodenleitung in Form und Anordnung mit den Ausnehmungen korrespondierende Vorsprünge eines Ringkontakts (oder des Stiftkontaktes oder beider) der Elektrodenleitung eingreifen. Durch eine nachfolgende Drehung der eingeführten Elektrodenleitung wird bewirkt, dass die Vorsprünge des Elektrodenleitungskontakts diejenigen der Anschlussbuchse hintergreifen. Sind insbesondere in axialer Reihung jeweils mehrere Kontakt- und Haltemittel vorgesehen, so greifen diese wechselweise in die zwischen ihnen vorhandenen Ausnehmungen ein. Das Ineinandergreifen der Vorsprünge schafft eine vergrößerte elektrische Kontaktfläche und einen ein Ausziehen der Elektrodenleitung aus der Anschlussbuchse sicher verhindernden Formschluss zwischen beiden. Die elektrischen und mechanischen Eigenschaften sind weiter verbessert, wenn die Nocken- bzw. Vorsprungsbereiche so angeordnet und ihre Eingriffsflächen so gestaltet sind, dass sie unter Erzeugung einer Flächenpressung in Wirkkontakt gelangen. Diese Flächenpressung verhindert einerseits in vorteilhafter Weise eine ungewollte Drehbewegung der Elektrodenleitung in eine Winkelstellung, in der die axiale Verriegelung aufgehoben wäre, und erbringt in vorteilhafter Weise einen hohen Kontaktdruck. In einer einfachen, aber wirkungsvollen Ausführung sind je drei oder vier, jeweils gleichartig ausgebildete, Kontakt- und Haltemittel gleichmäßig am Umfang der Anschlussbuchse bzw. des Ringkontakts verteilt - d.h. mit einem Winkelabstand ihrer Mittelebenen von 120° bzw. 90° - angeordnet.

Die Flanken der nockenförmig ausgebildeten Kontakt- und Haltemittel bildenden Seitenflächen sind jeweils in Umfangsrichtung gegeneinander geringfügig geneigt angeordnet, so dass die Nocken ein leicht keilförmiges Querschnittsprofil aufweisen. Dadurch wird auf einfache Weise einen hohe Flächenpressung erreicht und darüberhinaus bewirkt, dass das Eindrehen der Elektrodenleitung leicht auch aus einer Einsteckposition heraus erfolgen kann, in der die Winkellage des Steckers relativ zu Buchse nicht optimal ist.

Des weiteren wird durch die keilförmigen Querschnittsprofile der Kontakt- und Haltemittel eine Sicherheit gegen ein unbeabsichtigtes Überdrehen der Elektrodenleitung in eine Winkellage erreicht, in der die Kontakt- und Haltemittel nicht oder nur mit unzureichender Kontaktierung in Wirkungseingriff stehen würden.

Entsprechend einer günstigen Weiterbildung ist die Mittenebene der Nocken bzw. Vorsprünge der Elektrodenleitung relativ zur Längsache des Steckerstiftes (ähnlich den Schaufeln eines Lüfterrades) um einen Winkel von wenigen Grad geschwenkt. Die korrespondierenden Nocken bzw. Vorsprünge der Anschlussbuchse sind - in Umfangsrichtung betrachtet - beidseitig angefast. Auch dies ermöglicht in günstiger Weise ein Eindrehen der Elektrodenleitung auch bei nach dem Einstecken nicht exakt fluchtenden Vorsprüngen und Ausnehmungen.

Entsprechend einer Variante der in US 4 764 132 beschriebenen Anschlussbuchse sind an der Wandung der Anschlussbuchse sich in Umfangsrichtung erstreckende Federelemente vorgesehen, die jeweils einen Befestigungspunkt an der Hülse aufweisen. Die Federelemente unterliegen einer elastische Längung, wenn der Stift- oder Ringkontakt der Elektrodenleitung in die Anschlussbuchse eingeführt wird. Diese erbringt eine zur Erreichung einer hohen Auszugsicherheit gewünschte Flächenpressung mit dem Elektrodenleitungskontakt, ohne jedoch einen Formschluss herzustellen.

Bei einer derartigen Ausbildung der Anschlussbuchse ist keine Profilierung der Oberfläche des Ringkontakts am proximalen Ende der Elektrodenleitung durch zusätzliche Kontaktmittel vorgesehen. Der Durchmesser des Ringkontakts ist lediglich größer als der lichte Durchmesser der Anschlussbuchse (zwischen den ungelängten Federelementen) gewählt.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt.

Es zeigen:
- Figur 1: eine schematische Darstellung des Kopfteils eines Herzschrittmachers mit angeschlossener Elektrodenleitung,
- Figur 2: eine perspektivische Darstellung einer bevorzugten Ausführungsform der Erfindung,
- Figur 3: die Darstellung eines Schnittes längs der Linie A...A gemäß Figur 1,
- Figur 4: die Darstellung eines Schnittes längs der Linie B...B gemäß Figur 3,
- Figur 5: die Schnittdarstellung einer gegenüber Figur 4 modifizierten Weiterbildung der Erfindung,
- Figur 6: einen Teil-Längsschnitt durch ein Vergleichsbeispiel zum Vergleich mit der Erfindung,
- Figur 7: die Darstellung eines Schnittes längs der Linie C...C gemäß Figur 6,
- Figuren 8 und 9: die in den Figuren 6 und 7 gezeigte Hülse in zwei verschiedenen Herstellungsstufen,
- Figur 10: einen Teil-Längsschnitt durch ein weiteres Vergleichsbeispiel und
- Figur 11: die Darstellung eines Schnittes längs der Linie D...D gemäß Figur 10.

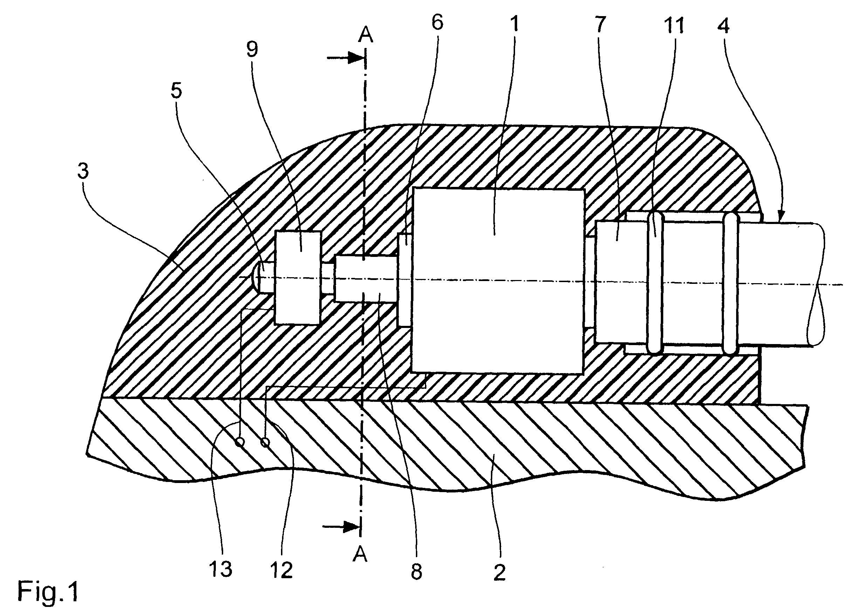

Figur 1 zeigt skizzenartig das Kopfteil 3 eines Herzschrittmachers 2, an den eine bipolare Elektrodenleitung 4 angeschlossen ist. Die am proximalen Ende der Elektrodenleitung befindlichen Kontakte 5 und 6 sind durch eine Isolierung 8 voneinander getrennt. Koaxial zum Stiftkontakt 5 ist ein Ringkontakt 6 der Elektrodenleitung 4 vorgesehen. Im Kopfteil 3 des Herzschrittmachers sind Anschlussbuchsen 1, 9 vorgesehen und jeweils einem der Kontakte 5, 6 der Elektrodenleitung 4 zugeordnet. Die elektrische Verbindung zwischen den Anschlussbuchsen 1, 9 und den (nicht dargestellten) Elekronik-Bausteinen des Schrittmachers 2 wird über interne Leitungen 12, 13 hergestellt. An der Außenisolation 7 der Elektrodenleitung 4 sind zwei Dichtringe 11 vorgesehen, welche bei implantiertem Herzschrittmacher ein Eindringen von Körperflüssigkeit über die Einstecköffnung des Kopfteils verhindern.

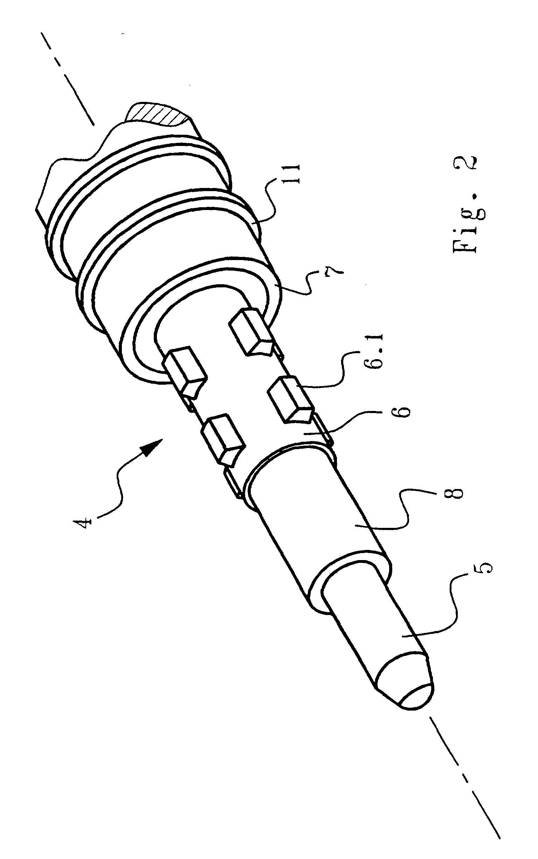

In Figur 2 ist das proximale Ende der Elektrodenleitung 4 in perspektivischer Darstellung gezeigt. Während der Stiftkontakt 5 eine glatte Oberfläche aufweist, sind an der Oberfläche des von dem Stiftkontakt 5 durch eine Isolierung 8 separierten Ringkontakts 6 der Leitung 4 eine Mehrzahl von als Pyramidenstumpf ausgebildeten und sich in radialer Richtung nach außen erstreckenden Stecker-Nocken 6.1 vorgesehen. Die auf einer Umfangslinie des Ringkontaktes gleichmäßig verteilt angeordneten Stecker-Nocken 6.1 bilden eine Nockengruppe, welche eine erste Segmentierung des Ringkontakts in Umfangsrichtung darstellt. Für eine zweite Segmentierung des Ringkontaktes 6 sind mehrere Nockengruppen vorgesehen, welche bei gleichem Abstand eine Reihung mit gleichen Abständen in axialer Richtung der Elektrodenleitung 4 aufweisen.

Die äußere Isolation 7 der Elektrodenleitung 4 trägt zwei Dichtungsringe 11, die die Einstecköffnung im Kopfteil des Schrittmachers feuchtigkeitsdicht verschließen.

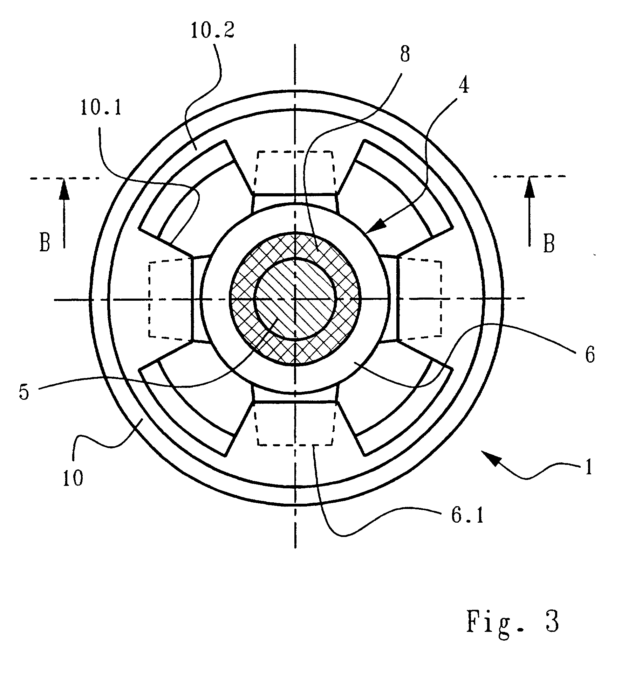

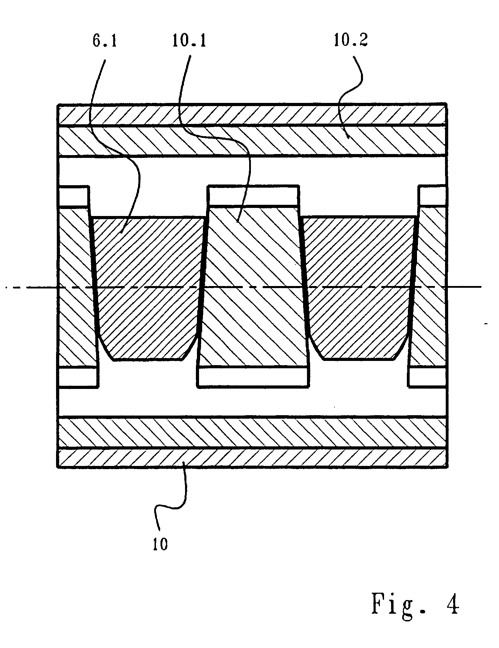

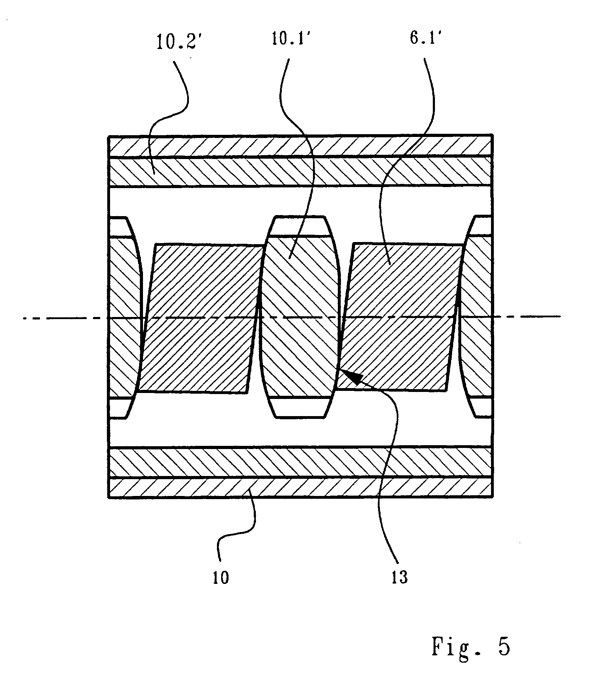

In Figur 3 ist eine aus der Anschlussbuchse 1 im Kopfteil des Herzschrittmachers und der Elektrodenleitung 4 gebildete Verbindungsanordnung in Form eines Schnittes längs der Linie A...A gemäß Figur 1 dargestellt, wobei die Elektrodenleitung 4 nach axialem Einschub in die Buchse 1 um 90° im Uhrzeigersinn um ihre Längsachse gedreht worden ist. Die Anschlussbuchse 1 besteht aus einer Außenhülse 10, in welche eine Innenhülse 10.2 eingesetzt ist. Die Innenhülse 10.2 weist an ihrer inneren Oberfläche eine Mehrzahl radial nach innen gerichteter Buchsennocken 10.1 auf, zwischen denen durch tangentiale Segmentierung gleichgroße Ausnehmungen vorgesehen sind. Durch eine Gruppierung von Buchsennocken 10.1 bei bestimmten Abstand zueinander in axialer Richtung ergeben sich Ausnehmungen (s. Figuren 4 und 5), deren Erstreckung in Umfangsrichtung auf die Abmessungen der Stecker-Nocken 6.1 an der fangsrichtung auf die Abmessungen der Stecker-Nocken 6.1 an der Peripherie des Ringkontaktes 6 derart abgestimmt ist, dass die Stecker-Nocken 6.1 beim Einschieben der Elektrodenleitung 4 in die Anschlussbuchse 1 axial in den Ausnehmungen bewegt werden können. Bei der nach dem Einschieben in die Anschlussbuchse auszuführenden Drehung der Elektrodenleitung 4 werden die Stecker-Nocken 6.1 in die in axialer Richtung vorhandenen Ausnehmungen zwischen den Buchsen-Nocken 10.1 der Anschlussbuchse 1 geschwenkt.

Hierdurch entsteht in günstiger Weise beim Eindrehen der Elektrodenleitung eine bajonettverschlussartige Verbindung zwischen den Stecker- und Buchsen-Nocken, welche einen ausreichenden Flächenkontakt bei gleichzeitig erhöhter Flächenpressung an den Randbereichen (vergleiche die Position 13 gemäß Figur 5) der Nockenflanken sichert. Dies sichert die Verbindungsanordnung gegen ein Lösen der Elektrodenleitung 4 bei mechanischen Belastung in axialer Richtung.

Vorteilhafte Ausführungsformen für die Stecker- und Buchsen-Nocken sind in den Figuren 4 und 5 als Ansicht eines Schnittes längs der Linie B...B gemäß Figur 3 dargestellt. Die Flanken (Seitenflächen) der einzelnen Nocken 6.1, 6.1', 10.1 und 10.1' sind grundsätzlich derart angeordnet bzw. ausgebildet, dass einerseits ein leichtes Eindringen der Stecker-Nocken 6.1, 6.1' des Ringkontaktes in die in axialer Richtung zwischen den Buchsen-Nocken 10.1, 10.1' an der Innenseite der Innenhülse 10.2, 10.2' der Anschlussbuchse vorhandenen Ausnehmungen beim Drehen der Elektrodenleitung ermöglicht wird. Andererseits wird diese Drehbewegung bei gleichzeitiger Arretierung der Stecker-Nocken 6.1, 6.1' begrenzt und bietet damit Schutz vor einem Überdrehen, bei dem die Kontaktierung nicht mehr gesichert ist. Der maximale Drehwinkel weist einen Wert von 90° auf.

Entsprechend Figur 4 sind sowohl die Flanken der Stecker-Nocken 6.1 als auch die Flanken der Buchsen-Nocken 10.1 gegenüber der tangentialen Richtung jeweils gegeneinander geneigt, so dass sich ein im wesentlichen keilförmiges Querschnittsprofil ergibt. Die einzelnen Keile weisen gegenüber der tangentialen Richtung einen gleichgroßen Öffnungswinkel auf. Dadurch ergibt sich nach erfolgter Drehung der Elektrodenleitung in vorteilhafter Weise eine kraft- und formschlüssige Verbindung zwischen den Kontaktmitteln bei großflächiger Kontaktierung und ausreichender Flächenpressung an den Flanken der Nocken.

Die Schnittdarstellung gemäß Figur 5 zeigt Stecker-Nocken 6.1' des Ringkontaktes der Elektrodenleitung, welche ein Querschnittsprofil in Form eines Parallelogramms aufweisen und gleichzeitig um ein geringes Maß um ihre sich radial erstreckende Längsachse geschwenkt sind. Die Flanken der Buchsen-Nocken 10.1' der Buchsenteile 10, 10.2' der Anschlussbuchse im Kopfteil des Herzschrittmachers (vergleiche die Positionen 1, 2 und 3 gemäß Figur 1) weisen beidseitig in tangentialer Richtung eine Anfasung auf, welche das Eindringen der Stecker-Nocken 6.1' in die zwischen den Buchsen-Nocken 10.1' in axialer Richtung befindlichen Ausnehmungen erleichtert, wenn die Stecker-Nocken 6.1' nicht genau mit den entsprechenden Ausnehmungen fluchten.

Nachfolgend sind Vergleichsbeispiele zum Vergleich mit der Erfindung beschrieben. Im Gegensatz zur Erfindung wird in den Vergleichsbeispielen kein formschlüssiger Eingriff hergestellt.

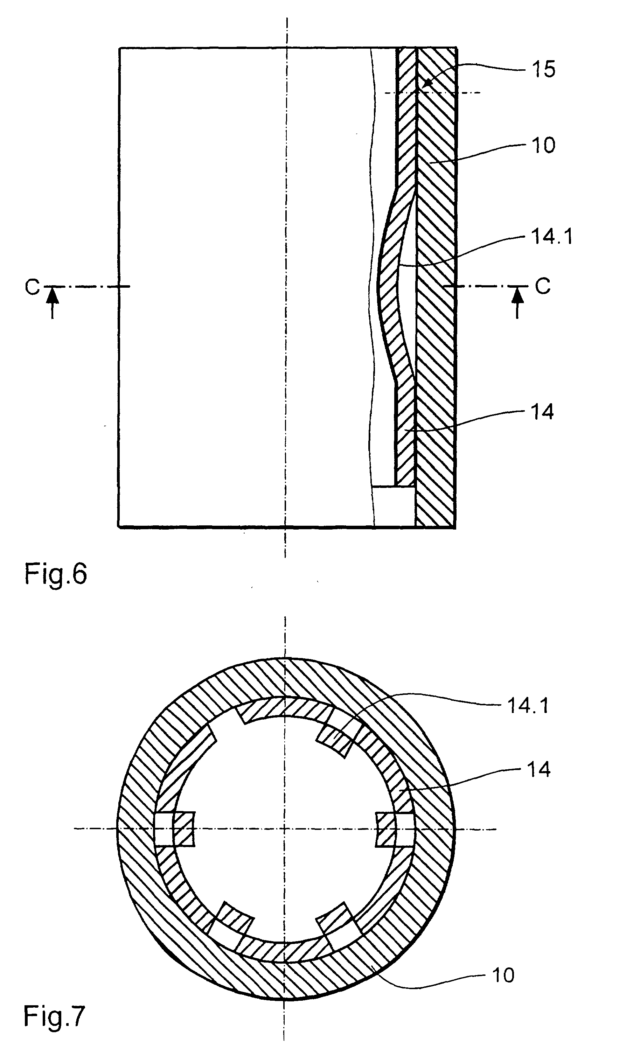

Die Darstellungen entsprechend den Figuren 6 und 7 zeigen einen Teil-Längsschnitt durch eine Anschlussbuchse 1 oder 9 bzw. eine Querschnittsansicht dieser Buchse (Schnitt längs der Linie C...C gemäß Figur 6). Die Anschlussbuchse weist in einer geschlossenen Außenhülse 10 eine Innenhülse 14 auf, an der streifenförmige Kontaktfederabschnitte 14.1 vorgesehen sind, die sich sowohl radial nach innen erstrecken als auch eine axiale Erstreckung aufweisen.

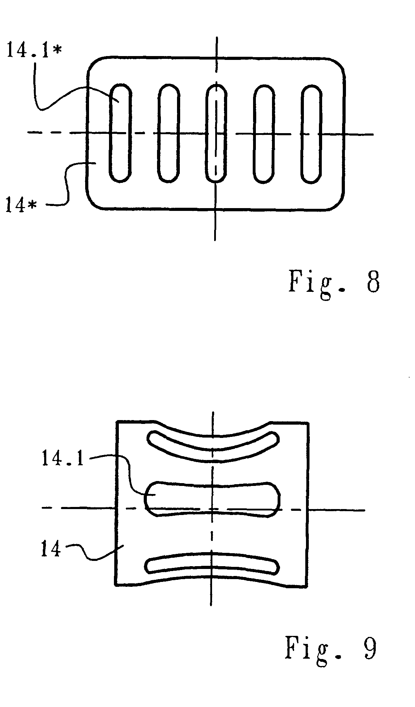

Die Figuren 8 und 9 zeigen die Innenhülse 14 in zwei Bearbeitungsstufen. Die Hülse 14 ist aus einem federnden Flachmaterial 14* gefertigt und wird durch einen Stanz- und Formgebungsvorgang in die erforderliche zylindrische Form gebracht, wobei gleichzeitig die Flächenbereiche 14.1* zu den Kontaktmitteln 14.1 ausgeprägt werden.

Die Innenhülse 14 ist - wie in Figur 6 gezeigt - durch mehrere, durch LaserSchweißen erzeugte Schweißpunkte 15 an einem ihrer Enden mit dem inneren Rand der Außenhülse 10 verbunden. Diese einseitige Befestigung sichert auf einfache Weise, dass sich die Hülse in axialer Richtung längen kann, wenn der glatte Stiftkontakt bzw. ein (abweichend von den vorstehend beschriebenen Ausführungen) glatt ausgeführter Ringkontakt des proximalen Endes der Elektrodenleitung in die Anschlussbuchse eingeführt wird. Dadurch wird die zur Kontaktierung erforderliche Federwirkung der Kontaktfederabschnitte 14.1 der Hülse 14 ständig aufrechterhalten.

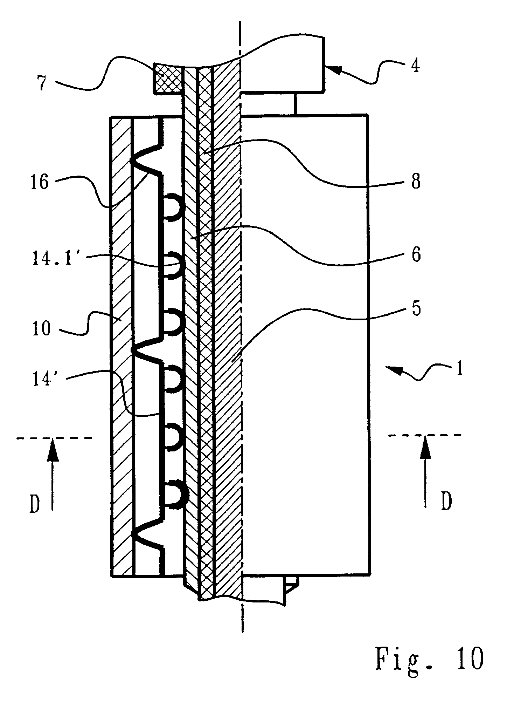

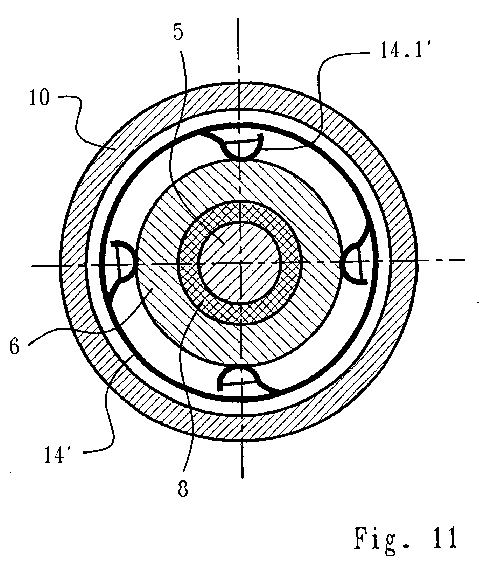

Die Figuren 10 und 11 zeigen als weitere Ausführungsform eine Anschlussbuchse 1 in Teil-Längsschnitt- bzw. als Querschnittsdarstellung längs der Linie D...D gemäß Figur 10. Das proximale Ende der Elektrodenleitung 4 ist in die Anschlussbuchse 1 eingeführt. Die in der Außenhülse 10 positionierte, aus Federblech gefertigte Innenhülse 14' weist mehrere radial nach außen weisende Ringwülste 16 auf, welche einen festen Sitz der Innenhülse 14' sichern. Die vier in der Schnittebene gemäß Figur 11 gleichmäßig am Hülsenumfang verteilten Kontaktfedern 14.1' sind streifenförmig ausgebildet und einseitig an der Hülsenwandung befestigt. Sie weisen in den Buchseninnenraum, wobei ihr freies Ende im wesentlichen halbkuglig ausgebildet ist und sie sich federnd auf dem Ringkontakt 6 abstützen. Die halbkuglige Form erleichert das Einschieben des zylindrischen Ringkontakts 6 der Elektrodenleitung 4. Die gereihte Anordnung der vorstehend beschriebenen Kontaktfedern in axialer Richtung der Anschlussbuchse 1 ermöglicht durch die entsprechende Anzahl von elektrischen Kontaktflächen und Kraftschlussbereichen eine sichere Kontaktierung des Ringkontakts und sichert einen festen Sitz des proximalen Endes der Elektrodenleitung im Kopfteil des Herzschrittmachers.

Die Erfindung beschränkt sich in ihrer Ausführung nicht auf die vorstehend angegebenen bevorzugten Ausführungsbeispiele. Vielmehr ist eine Vielzahl von Varianten denkbar, welche von der dargestellten Lösung auch bei abweichender Ausführung Gebrauch machen.

## Patentansprüche

1. Implantierbares Elektrostimulationsgerät (2) mit einem Kopfteil (3) zum elektrischen Anschluß einer Elektrodenleitung (4), das eine fest installierte Anschlußbuchse (1, 9) aufweist, an deren Innenwandung radial nach innen weisende Kontakt- und Haltemittel zum Eingriff mit einem Elektrodenleitungskontakt (5, 6) vorgesehen sind, wobei die Kontakt- und Haltemittel durch eine Mehrzahl von jeweils einen fest vorbestimmten Winkelabstand in Umfangsrichtung der Buchse und/oder axialen Abstand aufweisenden Nocken- oder Vorsprungsbereichen (10.1, 10.1') gebildet sind,
wobei die Nocken- oder Vorsprungsbereiche (10.1, 10.1') fest mit der Innenwandung verbunden oder aus dieser ausgeformt, sowie starr ausgebildet sind und dass die Nocken- oder Vorsprungsbereiche (10.1; 10.1') zum form- und vorzugsweise zusätzlich kraftschlüssigen Eingriff mit entsprechend angeordneten und geformten Bereichen (6.1) an einem Stift- oder Ringkontakt (5, 6) einer Elektrodenleitung (4) ausgebildet sind, und dass der formschlüssige und ggf. zusätzlich kraftschlüssige Eingriff durch eine Drehung des Stift- oder Ringkontakts (5, 6) um seine Längsachse herbeiführbar ist, wobei die Nocken- oder Vorsprungsbereiche der Anschlussbuchse ausgebildet sind, um von entsprechend angeordneten und geformten Bereichen (6.1) an einem Stift- oder Ringkontakt (5, 6) einer Elektrodenleitung (4) hintergriffen zu werden **dadurch gekennzeichnet, dass** die Nocken- oder Vorsprungsbereiche der Anschlußbuchse sich im wesentlichen radial erstreckende vordere und hintere Seitenflächen aufweisen, welche gegeneinander geneigt sind.

2. Elektrostimulationsgerät nach Anspruch 1, **dadurch gekennzeichnet, dass** die Nocken- oder Vorsprungsbereiche (10.1, 10.1') Eingriffsflächen aufweisen und die Nocken- oder Vorsprungsbereiche (10.1, 10.1') so angeordnet und ihre Eingriffsflächen so gestaltet sind, dass sie beim Eingriff mit den entsprechend angeordneten und geformten Bereichen (6.1, 6.1') des Stift- oder Ringkontakts (5, 6) unter Erzeugung einer Flächenpressung in Wirkkontakt geraten.

3. Elektrostimulationsgerät nach einem der vorangehenden Ansprüche, **dadurch gekennzeichnet, dass** die Nocken- und Vorsprungsbereiche (10.1, 10.1') ein im wesentlichen keilförmiges Querschnittsprofil besitzen.

4. Elektrostimulationsgerät einem der vorangehenden Ansprüche, **dadurch gekennzeichnet, daß** mindestens zwei Nocken- oder Vorsprungsbereiche (10.1; 10.1') bei übereinstimmender Lage in Umfangsrichtung in axialer Richtung gereiht sind.

5. Elektrostimulationsgerät nach einem der vorhergehenden Ansprüche, **dadurch gekennzeichnet, daß** mindestens drei Nocken- oder Vorsprungsbereiche (10.1, 10.1') jeweils in einer sich quer zur Längsachse der Anschlußbuchse (1) erstreckenden Ebene, insbesondere in gleichem Winkelabstand zueinander, angeordnet sind.

6. Elektrostimulationsgerät nach einem der vorangehenden Ansprüche, **dadurch gekennzeichnet, daß** die Anschlußbuchse (1) eine insgesamt aus einem leitfähigen Federmaterialblech geformte Innenhülse (10.2, 10.2') aufweist, welche die Nocken- oder Vorsprungsbereiche trägt.

7. Elektrodenleitung (4) für ein implantierbares Elektrostimulationsgerät (2), die einen Stift- oder Ringkontakt (5, 6) zur Einführung in eine Anschlußbuchse (1, 9) des Elektrostimulationsgeräts aufweist, wobei an der Oberfläche des Stift- oder Ringkontaktes (5, 6) radial nach außen weisende Kontakt- und Haltemittel vorgesehen sind, die durch eine Mehrzahl von fest mit der Oberfläche verbundenen oder aus dieser ausgeformten, jeweils einen fest vorbestimmten Winkelabstand in Umfangsrichtung des Stift- oder Ringkontaktes (5, 6) und/oder axialen Abstand aufweisenden, Nocken- oder Vorsprungsbereichen (6.1, 6.1') gebildet sind, die Nocken- oder Vorsprungsbereiche (6.1, 6.1') zum form- und vorzugsweise zusätzlich kraftschlüssigen Eingriff mit entsprechend angeordneten und geformten Bereichen (10.1, 10.1') der Anschlussbuchse (1) des Elektrostimulationsgerätes (2) ausgebildet sind und die Nocken- oder Vorsprungsbereiche (6.1, 6.1') derart ausgebildet sind, dass der formschlüssige und ggf. zusätzlich kraftschlüssige Eingriff durch eine Drehung des Stift- oder Ringkontakts (5, 6) um seine Längsachse herbeiführbar ist, wobei die Nocken- oder Vorsprungsbereiche (6.1, 6.1') der Elektrodenleitung ausgebildet sind, um entsprechend angeordnete und geformte Nocken- oder Vorsprungsbereiche (10.1, 10.1') der Anschlussbuchse zu hintergreifen **dadurch gekennzeichnet, dass** die Nocken- oder Vorsprungsbereiche (6.1, 6.1') der Elektrodenleitung im wesentlichen radial erstreckende Seitenflächen aufweisen, welche gegenüber der tangentialen Richtung geneigt sind.

8. Elektrodenleitung nach Anspruch 7, **dadurch gekennzeichnet, dass** die Nocken- oder Vorsprungsbereiche (6.1, 6.1') der Elektrodenleitung ein im wesentlichen keilförmiges Querschnittsprofil besitzen.

9. Elektrodenleitung nach Anspruch 7 oder 8, **dadurch gekennzeichnet, daß** mindestens zwei Nocken- oder Vorsprungsbereiche (6.1, 6.1') der Elektrodenleitung bei übereinstimmender Lage in Umfangsrichtung in axialer Richtung gereiht sind.

10. Elektrodenleitung nach einem der Ansprüche 7 bis 9, **dadurch gekennzeichnet, daß** die Nocken- oder Vorsprungsbereiche (6.1, 6.1') der Elektrodenleitung sich im wesentlichen radial erstreckende Seitenflächen aufweisen, welche gegeneinander geneigt sind.

11. Elektrodenleitung nach einem der Ansprüche 7 bis 10, **gekennzeichnet durch** eine Ausbildung ihres proximales Endes derart, daß sie in der Anschlußbuchse (3) des Elektrostimulationsgerätes (2) mittels eines **durch** vorbestimmte Axialverschiebung und Drehung des Endes um einen vorbestimmten Winkel erzeugten Kraft- und/oder Formschlusses zwischen ihren Kontakt- und Haltemitteln (6.1, 6.1') mit solchen in der Buchse (3), insbesondere **durch** Flächenpressung an deren jeweils benachbarten Seitenflächen, verbindbar ist.

## Claims

1. An implantable electrostimulation apparatus (2) having a head part (3) for electrical connection with an electrode lead (4), which comprises a securely installed connector socket (1, 9), at the inner wall of which radially inwardly pointing contact and retaining means are provided for engagement with an electrode lead contact (5, 6), wherein the contact and retaining means are formed by a number of lug or projection regions (10.1, 10.1') that comprise a predetermined angular spacing in the circumferential direction of the socket and/or axial spacing, wherein the lug or projection regions (10.1, 10.1') are securely connected to the inner wall or are formed from it, and also have a rigid construction and [in that] the lug or projection regions (10.1, 10.1') are constructed for positive and preferably additionally frictional engagement with corresponding disposed and formed regions (6.1) at a pin or ring contact (5, 6) of an electrode lead (4), and in that the positive and possibly frictional engagement can be produced by a rotation of the pin or ring contact (5, 6) around its longitudinal axis, wherein the lug or projection regions of the connector socket are constructed so that they can be grasped from behind by correspondingly disposed and formed regions (6.1) on a pin or ring contact (5, 6) of an electrode lead (4),
**characterised in that** the lug or projection regions of the connector socket comprise substantially radially extending front and rear side faces that are inclined towards each other.

2. An electrostimulation apparatus according to Claim 1,
**characterised in that** the lug or projection regions (10.1, 10.1') comprise engagement surfaces and the lug or projection regions (10.1, 10.1) are disposed and their engagement faces are shaped so that they come into effective contact upon engagement with the corresponding disposed and shaped regions (6.1, 6.1') of the pin or ring contact (5, 6) with the production of surface pressure.

3. An electrostimulation apparatus according to one of the preceding Claims,
**characterised in that** the lug and projection regions (10.1, 10.1') have a substantially wedge-shaped cross-sectional profile.

4. An electrostimulation apparatus according to one of the preceding Claims,
**characterised in that** at least two lug or projection regions (10.1; 10.1') are ranged side by side in the axial direction when their positions correspond in the circumferential direction.

5. An electrostimulation apparatus according to one of the preceding Claims,
**characterised in that** at least three lug or projection regions (10.1, 10.1') are disposed in each case in a plane extending transversely to the longitudinal axis of the connector socket (1), in particular with the same angular spacing.

6. An electrostimulation apparatus according to one of the preceding Claims,
**characterised in that** the connector socket (1) comprises an inner sleeve (10,2, 10.2'), which is formed overall from a conductive spring steel sheet material and bears the lug or projection regions.

7. An electrode lead (4) for an implantable electrostimulation apparatus (2), which comprises a pin or ring contact (5, 6) for introduction into a connector socket (1, 9) of the electrostimulation apparatus, wherein on the surface of the pin or ring contact (5, 6) are provided radially outwardly pointing contact and retaining means, which are formed by a number of lug or projection regions (6.1, 6.1') that are securely connected to the surface or are formed from it and in each case comprise a predetermined angular spacing in the circumferential direction of the pin or ring contact (5, 6) and/or axial spacing, the lug or projection regions (6,1, 6.1') are constructed for the positive and preferably additionally frictional engagement with correspondingly disposed and formed regions (10.1, 10.1') of the connector socket (1) of the electrostimulation apparatus (2) and the lug or projection regions (6.1, 6.1') are constructed in such a manner that the positive or possibly additionally frictional engagement can be produced by a rotation of the pin or ring contact (5, 6) around its longitudinal axis, wherein the lug or projection regions (6.1, 6.1') of the electrode lead are constructed in order to engage behind corresponding disposed and formed lug or projection regions (10.1, 10.1') of the connector socket,
**characterised in that** the lug or projection regions (6.1, 6.1') of the electrode lead comprise substantially radially extending side faces, which are inclined in relation to the tangential direction.

8. An electrode lead according to Claim 7,
**characterised in that** the lug or projection regions (6.1, 6.1') of the electrode lead have a substantially wedge-shaped cross-sectional profile.

9. An electrode lead according to Claim 7 or 8,
**characterised in that** at least two lug or projection regions (6.1, 6.1') of the electrode lead are line in the axial direction when their positions correspond in the circumferential direction.

10. An electrode lead according to one of Claims 7 to 9,
**characterised in that** the lug or projection regions (6.1, 6.1') of the electrode lead have substantially radially extending side faces, which are inclined towards one another.

11. An electrode lead according to one of Claims 7 to 10,
**characterised by** a formation of its proximal end in such a manner it can be connected in the connector socket (3) of the electrode stimulation apparatus (2) by means of a frictional and/or positive connection, produced by predetermined axial displacement and rotation of the end by a predetermined angle, between its contact and retaining means (6.1, 6.1') with said means in the socket (3), in particular by surface pressure at its respective adjacent side faces.

## Revendications

1. Appareil de stimulation implantable (2) muni d'une partie supérieure (3) en vue d'une connexion électrique d'un système d'électrodes (4), qui présente une douille de connexion (1, 9) installée fixement, à la paroi interne de laquelle sont prévus des moyens de contact et de retenue s'étendant radialement vers l'intérieur en vue d'un engrènement avec un contact de système d'électrodes (5, 6), où les moyens de contact et de retenue sont formés par une pluralité de zones de cames ou de parties saillantes (10.1, 10.1') présentant respectivement une distance angulaire prédéterminée dans la direction périphérique de la douille et/ou une distance axiale,
où les zones de cames ou de parties saillantes (10.1, 10.1') sont reliées à la paroi interne ou formées à partir de celle-ci, sont formées également de façon raide et en ce que les zones de cames ou de parties saillantes (10.1, 10.1') sont formées en vue d'un engrènement en plus par friction de façon mécanique et avantageuse avec des zones formées et disposées de façon correspondantes (6.1) à un contact à broche ou annulaire (5, 6) d'un système d'électrodes (4), et en ce que l'engrènement mécanique et éventuellement en plus par friction peut être provoqué par une rotation du contact à broche ou annulaire (5, 6) autour de son axe longitudinal, dans lequel les données de cames ou de parties saillantes de la douille de connexion sont formées, pour engrener avec des zones formées et disposées de façon correspondante (6.1) sur un contact à broche ou annulaire (5, 6) d'un système d'électrodes (4), **caractérisé en ce que** les zones de cames ou de parties saillantes de la douille de connexion présentent des surfaces latérales antérieure et postérieure s'étendant sensiblement radialement, qui sont inclinées l'une vers l'autre.

2. Appareil de stimulation selon la revendication 1, **caractérisé en ce que** les zones de cames ou de parties saillantes (10.1, 10.1') présentent des surfaces d'engrènement et les zones de cames ou de parties saillantes (10.1, 10.1') sont disposées et leurs surfaces d'engrènement sont formées de sorte qu'elles viennent en contact efficace lors de l'engrènement avec les zones formées et disposées de façon correspondante (6.1, 6.1') du contact à broche ou annulaire (5, 6) lors de la génération d'une pression superficielle.

3. Appareil de stimulation selon l'une des revendications précédentes, **caractérisé en ce que** les zones de cames et de parties saillantes (10.1, 10.1') ont un profil en coupe sensiblement cunéiforme.

4. Appareil de stimulation selon l'une des revendications précédentes, **caractérisé en ce qu'**au moins deux zones de cames ou de parties saillantes (10.1, 10.1') se suivent dans une position prédéterminée dans une direction périphérique dans une direction axiale.

5. Appareil de stimulation selon l'une des revendications précédentes, **caractérisé en ce qu'**au moins trois zones de cames ou de parties saillantes (10.1, 10.1') sont disposées respectivement dans un plan s'étendant à travers par rapport à l'axe longitudinal de la douille de connexion (1), en particulier à la même distance angulaire.

6. Appareil de stimulation selon l'une des revendications précédentes, **caractérisé en ce que** la douille de connexion (1) présente un manchon interne (10.1, 10.2') formé dans son ensemble à partir d'une tôle élastique conductrice, qui support les zones de cames ou de parties saillantes.

7. Systèmes d'électrodes (4) pour un appareil de stimulation implantable (2), qui présente un contact à broche ou annulaire (5, 6) pour une insertion dans une douille de connexion (1, 9) de l'appareil de stimulation, dans lequel sont prévus à la surface supérieure du contact à broche ou annulaire (5, 6) des moyens de contact et de retenue s'étendant radialement vers l'extérieur, qui sont formés par une pluralité de zones de cames ou de parties saillantes (6.1, 6.1') présentant respectivement une distance angulaire prédéterminée dans la direction périphérique du contact à broche ou annulaire (5, 6) et/ou une distance axiale, reliées à la surface supérieure ou formées à partir de celle-ci, les zones de cames ou de parties saillantes (6.1, 6.1') sont formées en vue d'un engrènement en plus par friction de façon mécanique et avantageuse avec des zones formées et disposées de façon correspondante (10.1, 10.1') de la douille de connexion (1) de l'appareil de stimulation (2) et les zones de cames ou de parties saillantes (6.1, 6.1') sont formées de sorte que, l'engrènement mécanique et éventuellement en plus par friction peut être provoqué par une rotation du contact à broche ou annulaire (5, 6) autour de son axe longitudinal, dans lequel les zones de cames ou de parties saillantes (6.1, 6.1') du système d'électrodes sont formées, pour engrener avec les zones de cames ou de parties saillantes formées et disposées de façon correspondante (10.1, 10.1') de la douille de connexion, **caractérisé en ce que** les zones de cames ou de parties saillantes (6.1, 6.1') du système d'électrodes présentent des surfaces latérales s'étendant sensiblement radialement, qui sont inclinées dans la direction tangentielle l'une contre l'autre.

8. Système d'électrodes selon la revendication 7, **caractérisé en ce que** les zones de cames ou de parties saillantes (6.1, 6.1') du système d'électrodes ont un profil en coupe sensiblement cunéiforme.

9. Système d'électrodes selon la revendication 7 ou 8, **caractérisé en ce qu'**au moins deux zones de cames ou de parties saillantes 56.1, 6.1') du système d'électrodes se suivent dans une position prédéterminée dans une direction périphérique dans une direction axiale.

10. Système d'électrodes selon l'une des revendications 7 à 9, **caractérisé en ce que** les zones de cames ou de parties saillantes (6.1, 6.1') du système d'électrodes présentent des surfaces latérales s'étendant sensiblement radialement, qui sont inclinées l'une contre l'autre.

11. Système d'électrodes selon l'une des revendications 7 à 10, **caractérisé par** une réalisation de leur extrémité proximale de sorte qu'il peut être relié dans la douille de connexion (3) de l'appareil de stimulation (2) au moyen d'une connexion mécanique et/ou par friction générée par un déplacement axial prédéterminé et une rotation de l'extrémité d'un angle prédéterminé entre leurs moyens de contact et de retenue (6.1, 6.1') avec ces surfaces latérales respectivement voisines dans la douille (3), en particulier par pression superficielle sur celles-ci.
